# EUROPÄISCHE PATENTSCHRIFT

(11) **EP 2 239 246 B1**
(45) Veröffentlichungstag und Bekanntmachung des Hinweises auf die Patenterteilung: **13.04.2016**
(21) Anmeldenummer: 10168296.1
(22) Anmeldetag: 04.02.2000
(51) Int. Cl.: C04B 41/82, C04B 41/88, A61K 6/06

(54) **EINFÄRBUNG VON KERAMIKEN MITTELS IONISCHER ODER KOMPLEXHALTIGER LÖSUNGEN**
COLORING CERAMICS BY WAY OF IONIC OR COMPLEX-CONTAINING SOLUTIONS
COLORATION DE CERAMIQUES AU MOYEN DE SOLUTIONS IONIQUES OU RENFERMANT DES COMPLEXES

(30) Priorität: 04.02.1999 DE 19904522
(43) Veröffentlichungstag der Anmeldung: 13.10.2010
(62) Teilanmeldung aus: 00905025.3
(73) Patentinhaber: 3M Deutschland GmbH, 41453 Neuss (DE)
(72) Erfinder: Suttor, Daniel, 70599 Stuttgart (DE); Hauptmann, Holger, 82404 Sindelsdorf (DE); Schnagl, Robert, 86860 Jengen (DE); Schmittner, Sybille, 82266 Inning (DE)
(74) Vertreter: Vossius & Partner Patentanwälte Rechtsanwälte mbB

(56) Entgegenhaltungen:
- EP-A1- 0 329 565
- DE-A1- 3 424 777

## Beschreibung

Die Erfindung betrifft die Einfärbung von Keramiken mittels ionischer oder komplexhaltiger Lösungen. Im besonderen betrifft die Erfindung die Einfärbung von Dentalkeramiken auf Zirkonoxidbasis mittels Lösungen von Seltenerdenmetallen und Nebengruppenelementen.

Keramiken werden aufgrund ihrer physikalischen Eigenschaften bei der Erstellung von hochwertigem Zahnersatz sehr geschätzt. Aluminium- und Zirkonoxidkeramiken sind im Medizinbereich seit langem die Materialien der Wahl (Sonderdruck aus Industrie Diamanten Rundschau, IDR 2/1993, "Aluminium- und Zirkonoxidkeramik in der Medizin"). Im Dentalbereich gibt es eine Vielzahl von Publikationen, die sich mit der Verwendung von Keramiken zur Herstellung von Prothesen beschäftigen. Ebenso sind diverse Keramiksysteme bereits auf dem Dentalmarkt verfügbar (CEREC, Fa. Siemens; Procera, Fa. Nobel-Biocare).
EP-A 329565 betrifft eine Klammer aus Keramik und ihr Herstellungsverfahren.

Insbesondere im Dentalbereich spielen aber nicht nur die mechanischen Parameter eine große Rolle, sondern besonders auch die Ästhetik. Transluzenz und Farbgebung der Gerüst- oder Verblendkeramiken sind wichtig, um dem Patienten ein ein natürliches Aussehen seines künstlichen Zahnersatzes zu ermöglichen.

Zahnersatz wird üblicherweise aus einem Gerüst und einer Verblendung hergestellt.

Bei den bisher bekannten Systemen kann nur eine oberflächliche individuelle Einfärbung des Grundgerüstes durch den Zahntechniker vorgenommen werden, dabei sind die ästhetischen Gestaltungsmöglichkeiten eingeschränkt.

Um ein natürliches Erscheinungsbild der Prothese zu erzielen, muß die Zahnfarbe und die Transluzenz über mehrere Schichten hinweg, beginnend mit dem Gerüst, simuliert werden.

Das natürliche Erscheinungsbild einer Prothese wird gewährleistet durch eine möglichst hohe freie Weglänge z = x + y + m des einfallenden Lichtes durch die Schicht (x) der Verblendkeramik und Schicht (m) der Gerüstkeramik und ggf. einer Zwischenschicht (y).

Herkömmliche Systeme müssen zur Veränderung des Grundfarbtones der Gerüstkeramik mit färbenden Zwischenschichten, beispielsweise Opaquer-Linern, arbeiten, die keine oder stark verringerte Transluzenz aufweisen; die freie Weglänge des Lichtes verringert sich um die Dicke der Gerüstkeramik (m) und der Zwischenschicht (y) auf z = x. Eine Beschreibung dieser Vorgehensweise ist z. B. in den Gebrauchsinformationen der Fa. Vita zum System Vita-Dur α oder der Fa. DUCERA mit dem System ALL Ceram zu finden.

Derartige Systeme verwenden als Zwischenschicht Farbpasten bzw. Farbsuspensionen, die in mehreren Arbeitsgängen vom Zahntechniker auf das Gerüst aufgebracht und abschließend im Ofen gebrannt werden.

Dieser Vorgang ist nicht nur zeitaufwendig, sondern auch kostenintensiv.

Der Erfindung liegt somit die Aufgabe zugrunde, eine gefärbte Dentalkeramik, inbesondere keramischen Zahnersatz, bereitzustellen, das eine optimale Ästhetik bei minimalem Arbeitsaufwand und bei minimierten Kosten gewährleistet.

Überraschend wird diese Aufgabe gelöst durch die Bereitstellung von gefärbter Dentalkeramik erhältlich durch ein Verfahren zum Einfärben von Keramiken im porösen oder saugfähigen Zustand, das dadurch gekennzeichnet ist, daß die Keramiken transluzent sind und zum Einfärben Metallionen-Lösungen oder Metallkomplex-Lösungen verwendet werden. Hierfür bevorzugte Lösungen enthalten definierte Konzentrationen mindestens eines der Salze oder Komplexe der Seltenerden-Elemente oder der Elemente der Nebengruppen.

Die Lösungen sind vorzugsweise auf wäßriger oder alkoholischer Basis. Geeignete Salze oder Komplexe sind bevorzugt solche aus der Gruppe der Seltenerden oder der II. oder VIII. Nebengruppe, insbesondere Pr, Er, Fe, Co, Ni, Cu.

Bevorzugt sind Salze oder Komplexe mit anorganischen Gegenionen wie z. B. Cl⁻, Br⁻, J⁻, SO₄²⁻, SO₃²⁻, NO₂⁻, NO₃⁻, ClO₄⁻, ONC⁻, SCN⁻ wobei auch Oxokomplexe saurer oder basischer Salze gemeint sein können, nicht aber Doppelsalze mit einem Element der 1. oder 2. Hauptgruppe. Desweiteren sind Salze oder Komplexe mit organischen Ionen oder Liganden bevorzugt, die 1 bis 30 C-Atome und eine Anzahl von 1 bis 10 Heteroatome, wie O, N, S, enthalten. im einzelnen sind dies Alkoxide oder Salze organischer Säuren. Bevorzugt sind hier unter den Alkoxiden die Salze der C₁-C₁₀-Alkanole, insbesondere die Methoxide, Ethoxide, n- und i-Propoxide und n-, i-, sek- bzw. tert-Butoxide. Unter den Salzen organischer Säuren sind diejenigen von Mono-, Di- und Tri- C₁-C₂₀-Carbonsäuren bevorzugt, insbesondere Formiat, Acetat, Malat, Maleat, Maleinat, Tartrat, Oxalat. Zuletzt sind auch Komplexbildner unter den Liganden zu verstehen, die dazu dienen, die Metallsalze in ihrer Oxidationsstufe und in Lösung zu stabilisieren. Diese können organische C₂-C₂₀-Moleküle mit bis zu 10 Heteroatomen O, N oder S, darunter insbesondere EDTA und seine Salze, NTA, Salicylsäure, Phenole, 5-Sulfosalicylsäure etc., sein.

Bevorzugt sind wäßrige oder alkoholische Lösungen von Pr, Er, Fe, beispielsweise als Chloride, Acetate oder Alkoholate.

Die Ionen oder Komplexe werden vorzugsweise in Konzentrationen von 0,0001 bis 15 Gew.-%, besonders bevorzugt von 0,001 bis 10 Gew.-% und ganz besonders bevorzugt von 0,01 bis 7 Gew.-% eingesetzt.

Unter Keramiken und Dentalkeramiken werden hier alle hochfesten Oxide der Elemente der Hauptgruppen II, III und IV und der Nebengruppen III und IV sowie deren Mischungen verstanden, insbesondere Al₂O₃, ZrO₂, sowohl teil- als auch volistabilisiert, MgO, TiO₂ und deren Mischungen. Insbesondere werden unter Keramiken und Dentalkeramiken transluzente Keramiken verstanden.

Überraschend hierbei ist ferner, daß die Farbtiefe der Einfärbung nicht von der Einwirkdauer der Lösung, sondern nur von deren Konzentration abhängig ist. Dies ist besonders vorteilhaft, da der Zahntechniker nicht auf sekundengenaue Einwirkzeiten fixiert ist, sondern seine Arbeiten innerhalb gewisser Toleranzen beliebig lange mit den erfindungsgemäßen Lösungen behandeln kann. Die Einwirkdauer der Lösung kann theoretisch beliebig lange sein. Sie ist nur abhängig von anderen Effekten in der Lösung, beispielsweise pH-Wert-Änderungen oder Freisetzung von Ionen, die den Färbevorgang behindern können. So ergibt sich im allgemeinen eine Einwirkdauer, bis zu der die Farbtiefe der Einfärbung sich nicht verändert, von einigen Stunden. Die Einwirkdauer beträgt vorzugsweise unter 2 Stunden, insbesondere unter 1 Stunde und besonders bevorzugt unter 20 Minuten.

Vorteilhafterweise kann durch die vorliegende Erfindung die oben erwähnte Zwischenschicht (y) komplett entfallen, da bereits die Gerüstkeramik durch die erfindungsgemäßen Lösungen individuell eingefärbt werden kann. Es entfällt daher ein zusätzlicher kosten- und zeitintensiver Schritt des Aufbrennens der Zwischenschicht. Dem einfat!enden Licht steht nun die freie Weglänge z = x + y + m zur Verfügung, da der Weg nicht mehr durch die Zwischenschicht unterbrochen wird.

Die erfindungsgemäßen Lösungen können neben den Salzen oder Komplexen der Seltenerden-Elemente oder der Nebengruppenelemente auch Stabilisierungsmittel, wie Komplexbildner, Malhilfsmittel sowie organische Farbpigmente zur Erleichterung der Farbabstimmung durch den Zahntechniker enthalten.

Als Stabilisierungsmittel geeignet sind Komplexbildner, wie Ethylendiamintetraessigsäure. Unter Malhilfsmitteln sind beispielsweise temporäre Bindemittel und Thixotropiemittel, wie Polyglykole, Polysaccharide, Polyethylenglykole, Polyvinylalkohole, hydrierte Rizinusöle, zu verstehen.

Aufgrund der niedrigen Konzentrationen an farbgebenden ionen oder Komplexen innerhalb der erfindungsgemäßen Lösungen und der damit verbundenen schlechten optischen Erkennbarkeit des aufgebrachten Farbtons, können auch organische Pigmente zur Erleichterung der Farbabstimmung durch den Zahntechniker zugesetzt werden. Besonders hilfreich sind diese Zusätze bei der bereichsweisen Applikation der Lösungen über Applikationsinstrumente. Die Zusätze sind so zu wählen, daß sie beim Brennen der prothetischen Arbeit rückstandsfrei zerstört werden.

Die erfindungsgemäßen Lösungen können auf folgende Weisen auf die vorgesinterten bzw. saugfähigen Keramiken aufgebracht werden:
1. Tauchen der Keramik in Lösungen definierter Konzentrationen;
2. Auftragen der Lösungen auf die Keramik mittels geeigneter Applikationsinstrumente, beispielsweise Pinsel, Tupfer;
3. Auftragen der Lösungen auf die Keramik mittels Sprühverfahren.

Mit dem erfindungsgemäßen Verfahren können Wandstärken von bis zu 10 mm, bevorzugt 7 mm, durchgehend eingefärbt werden. Insbesondere im dentalen Bereich bei der Herstellung von Kronen und Brücken sind Abmessungen von 10 mm, bevorzugt 7 mm, für den Durchmesser eines Werkstückes und 7 mm, bevorzugt 5 mm, für die Höhe eines Werkstückes möglich. Diese mm-Angaben beziehen sich auf die Dicken der einfärbbaren Wandstärken der dentalen Werkstücke. Natürlich sind auch Werkstücke außerhalb der hier angegebenen Grenzen im Umfang der Erfindung enthalten.

Vorzugsweise werden die Keramiken vollständig durchgefärbt.

Die Anmeldung beschreibt auch einen Kit, umfassend
(i) mindestens eine Vorratsflasche mit einer Metallionen- oder Metallkomplex-Lösung für das Einfärben von Keramiken,
(ii) ein Behältnis für das Einfärben, und
(iii) gegebenenfalls ein Sieb.

Im Nachstehenden werden bevorzugte Verfahren beschrieben, welche zur Einfärbung von Keramiken im porösen oder saugfähigen Zustand geeignet sind:
1. Verfahren zum Einfärben von Keramiken im porösen oder saugfähigen Zustand, dadurch gekennzeichnet, daß die Keramiken transluzent sind und zum Einfärben Metallionen-Lösungen oder Metallkomplex-Lösungen verwendet werden.
2. Verfahren nach Punkt 1, dadurch gekennzeichnet, daß Dentalkeramiken eingefärbt werden.
3. Verfahren nach Punkt 1 oder 2, dadurch gekennzeichnet, daß die Lösungen mindestens eines der Ionen oder Komplexe der Seltenerden-Elemente oder Nebengruppen enthalten.
4. Verfahren nach Punkt 3, dadurch gekennzeichnet, daß die Lösungen Pr, Er, Fe, Co, Ni oder Cu enthalten.
5. Verfahren nach Punkt 3 oder 4, dadurch gekennzeichnet, daß als Salze Chloride, Acetate oder Alkohole sowie Oxokomplexe verwendet werden.
6. Verfahren nach mindestens einem der Punkte 1 bis 5, dadurch gekennzeichnet, daß Dentalkeramiken im vorgesinterten Zustand verwendet werden.
7. Verfahren nach mindestens einem der Punkte 1 bis 6, dadurch gekennzeichnet, daß Dentalkeramiken auf Zirkonoxid- oder Aluminiumoxidbasis verwendet werden.
8. Verfahren nach mindestens einem der Punkte 1 bis 7, dadurch gekennzeichnet, daß die ionischen oder komplexhaltigen Lösungen auf wäßriger oder alkoholischer Basis sind.
9. Verfahren nach mindestens einem der Punkte 1 bis 8, dadurch gekennzeichnet, daß die Einwirkdauer der ionischen oder komplexhaltigen Lösungen im Stundenbereich, insbesondere unter 2 Stunden, ganz besonders unter 1 Stunde und besonders bevorzugt unter 20 Minuten liegt.
10. Verfahren nach mindestens einem der Punkte 1 bis 9, dadurch gekennzeichnet, daß die Konzentration der Lösungen 0,001 bis 15 Gew.-% beträgt.
11. Verfahren nach mindestens einem der Punkte 1 bis 10, dadurch gekennzeichnet, daß das Einfärben durch Tauchen der Keramik in die Lösungen, durch Auftragen der Lösungen auf die Keramik mit Hilfe von Applikationsinstrumenten oder durch Aufsprühen der Lösungen auf die Keramik erfolgt.
12. Verfahren nach mindestens einem der Punkte 1 bis 11, dadurch gekennzeichnet, daß die einzufärbenden Keramiken einen Durchmesser von 10 mm, vorzugsweise 7 mm, und eine Höhe von 7 mm, vorzugsweise 5 mm, besitzen.
13. Verfahren nach mindestens einem der Punkte 1 bis 12, dadurch gekennzeichnet, daß die Keramiken vollständig durchgefärbt werden.

Die Erfindung wird nachfolgend durch Beispiele näher erläutert, ohne daß sie dadurch beschränkt sein soll.

### Konzentrationsabhängige Einfärbung von durch 3 Mol Yttriumoxid stabilisiertem Zirkonoxid

Zur Herstellung der Lösungen werden die entsprechenden Mengen Farbreagenz in Wasser gelöst. Keramikkörper werden darin 5 min tauchgebadet und anschließend getrocknet und gesintert. Die Proben werden anschließend für die Farbmessung geschliffen und poliert. Der Farbbestimmung liegen folgende Parameter zugrunde:
- Opazitätswert O:: Maß für die Transparenz (0% ist voll transparent, 100% ist opak);
- L*-Wert:: Helligkeit (100: vollständige Reflexion, 0: keine Reflexion);
- a*-Wert:: Rot-Grünverschiebung (+a: rot; -a: grün);
- b*-Wert:: Gelb-Blauverschiebung (+b: gelb; -b: blau);
Meßgerät: Fa. HunterLab, LabScan Spectrocolorimeter;
Meßmethode: Cielab (Farbe); Opazität nach ASTM D2805 / TAPPI T425 / TAPPI T519.

Zum Nachweis der Unabhängigkeit der Farbintensität von der Einwirkdauer der Lösung werden bei fester Lösungskonzentration verschiedene Einwirkzeiten zugrundegelegt und die Farbbestimmung analog durchgeführt.

Als Material wurde käufliche Zirkonoxidkeramik der Firma Tosoh, Japan vom Typ TZ3YE verwendet.

**Einfärbung mit Fe(III)Cl₃-Lösungen**

| **Konzentration Lösung [Gew.-%]** | **L*** | **a*** | **b*** | **O** |
|---|---|---|---|---|
| | | | | |
| **0** | 85,67 | -0,97 | 1,51 | 91,4 |
| **0,1** | 83,93 | -1,67 | 5,15 | 92,36 |
| **0,3** | 79,04 | -1,52 | 22,35 | 95,1 |
| **0,5** | 75,37 | 1,16 | 25 | 95,32 |
| **0,75** | 74,01 | 1,72 | 25,91 | 96,51 |
| **1** | 72,25 | 2,83 | 24,67 | 97,79 |

**Einfärbung mit Pr(III)Acetat-Lösungen**

| **Konzentration Lösung [Gew.-%]** | **L*** | **A*** | **b*** | **O** |
|---|---|---|---|---|
| | | | | |
| **0,1** | 81,02 | -3,60 | 24,98 | 89,98 |
| **0,25** | 80,80 | -3,02 | 34,17 | 91,40 |
| **0,75** | 74,85 | 4,77 | 47,31 | 92,11 |

| | | | | |
|---|---|---|---|---|
| **Ergebnis:** Über die Konzentration der Lösung kann die Intensität der Farbe gesteuert werden. | | | | |

**Abhängigkeit der Farbintensität von der Einwirkdauer**

**Lösungskonzentration: 0,75 Gew.-% Fe (III) Cl - Lösung**

| **Einwirkdauer** | **L*** | **a*** | **b*** | **O** |
|---|---|---|---|---|
| | | | | |
| **2 Minuten** | 75,18 | 0,32 | 20,15 | 96,05 |
| **5 Minuten** | 76,06 | -0,42 | 21,4 | 95,86 |
| **10 Minuten** | 75,18 | -0,09 | 22,4 | 96,08 |
| **20 Minuten** | 75,80 | -0,21 | 23,11 | 96,37 |

| | | | | |
|---|---|---|---|---|
| **Ergebnis:** Die Einwirkdauer hat keinen Einfluß auf die Farbintensität. | | | | |

## Patentansprüche

1. Gefärbte Dentalkeramik erhältlich durch ein Verfahren zum Einfärben von Dentalkeramiken im porösen oder saugfähigen Zustand, **dadurch gekennzeichnet, dass** die Dentalkeramiken transluzent sind und zum Einfärben Metallionen-Lösungen oder Metallkomplex-Lösungen verwendet werden.

2. Gefärbte Dentalkeramik nach Anspruch 1, **dadurch gekennzeichnet, dass** die Lösungen mindestens eines der Ionen oder Komplexe der Seltenerden-Elemente oder Nebengruppen enthalten.

3. Gefärbte Dentalkeramik nach Anspruch 2, **dadurch gekennzeichnet, dass** die Lösungen Pr, Er, Fe, Co, Ni oder Cu enthalten.

4. Gefärbte Dentalkeramik nach Anspruch 2 oder 3, **dadurch gekennzeichnet, dass** die Lösungen Salze enthalten und als Salze Chloride, Acetate oder Alkohole sowie Oxokomplexe verwendet werden.

5. Gefärbte Dentalkeramik nach mindestens einem der Ansprüche 1 bis 4, **dadurch gekennzeichnet, dass** Dentalkeramiken im vorgesinterten Zustand verwendet werden.

6. Gefärbte Dentalkeramik nach mindestens einem der Ansprüche 1 bis 5, **dadurch gekennzeichnet, dass** Dentalkeramiken auf Zirkonoxid- oder Aluminiumoxidbasis verwendet werden.

7. Gefärbte Dentalkeramik nach mindestens einem der Ansprüche 1 bis 6, **dadurch gekennzeichnet, dass** die ionischen oder komplexhaltigen Lösungen auf wässriger oder alkoholischer Basis sind.

8. Gefärbte Dentalkeramik nach mindestens einem der Ansprüche 1 bis 7, **dadurch gekennzeichnet, dass** die Einwirkdauer der ionischen oder komplexhaltigen Lösungen im Stundenbereich, insbesondere unter 2 Stunden, ganz besonders unter 1 Stunde und besonders bevorzugt unter 20 Minuten liegt.

9. Gefärbte Dentalkeramik nach mindestens einem der Ansprüche 1 bis 8, **dadurch gekennzeichnet, dass** die Konzentration der Lösungen 0,001 bis 15 Gew.-% beträgt.

10. Gefärbte Dentalkeramik nach mindestens einem der Ansprüche 1 bis 9, **dadurch gekennzeichnet, dass** das Einfärben durch Tauchen der Dentalkeramik in die Lösungen, durch Auftragen der Lösungen auf die Dentalkeramik mit Hilfe von Applikationsinstrumenten oder durch Aufsprühen der Lösungen auf die Dentalkeramik erfolgt.

11. Gefärbte Dentalkeramik nach mindestens einem der Ansprüche 1 bis 10, **dadurch gekennzeichnet, dass** die Wandstärke der einzufärbenden Dentalkeramiken einen Durchmesser von bis zu 10 mm, vorzugsweise bis zu 7 mm, besitzen.

12. Gefärbte Dentalkeramik nach mindestens einem der Ansprüche 1 bis 11, **dadurch gekennzeichnet, dass** die Dentalkeramiken vollständig durchgefärbt werden.

13. Gefärbte Dentalkeramik nach mindestens einem der Ansprüche 1 bis 12, **dadurch gekennzeichnet, dass** die Dentalkeramiken nach dem Einfärben gebrannt werden.

14. Gefärbte Dentalkeramik nach mindestens einem der Ansprüche 1 bis 13, **dadurch gekennzeichnet, dass** es sich bei den Dentalkeramiken um Kronen oder Brücken handelt.

## Claims

1. Colored dental ceramic, obtainable through a method for the coloration of dental ceramics in the porous or absorbent state, **characterized in that** the dental ceramics are translucent and for the coloration metal ion solutions or metal complex solutions are used.

2. Colored dental ceramic according to claim 1, **characterized in that** the solutions contain at least one of the ions or complexes of the rare earth elements or subgroups.

3. Colored dental ceramic according to claim 2, **characterized in that** the solutions contain Pr, Er, Fe, Co, Ni or Cu.

4. Colored dental ceramic according to claim 2 or 3, **characterized in that** the solutions contain salts, and **in that** chlorides, acetates or alcohols, as well as oxo complexes, are used as salts.

5. Colored dental ceramic according to at least one of claims 1 to 4, **characterized in that** dental ceramics are used in the pre-sintered state.

6. Colored dental ceramic according to at least one of claims 1 to 5, **characterized in that** dental ceramics are used that are based on zirconium oxide or aluminum oxide.

7. Colored dental ceramic according to at least one of claims 1 to 6, **characterized in that** the ionic or complex-containing solutions are aqueous or alcohol-based.

8. Colored dental ceramic according to at least one of claims 1 to 7, **characterized in that** the exposure time of the ionic or complex-containing solutions is in the hour range, in particular under 2 hours, most particularly under 1 hour and particularly preferably under 20 minutes

9. Colored dental ceramic according to at least one of claims 1 to 8, **characterized in that** the concentration of the solution is 0.001 to 15 % by weight.

10. Colored dental ceramic according to at least one of claims 1 to 9, **characterized in that** coloring is accomplished by immersing the dental ceramic in the solution, by applying the solution to the dental ceramic using application instruments or by spraying the solution onto the dental ceramic.

11. Colored dental ceramic according to at least one of claims 1 to 10, **characterized in that** the wall thickness of the dental ceramic to be colored has a diameter of up to 10 mm, preferably up to 7 mm.

12. Colored dental ceramic according to at least one of claims 1 to 11, **characterized in that** the dental ceramics are completely through-colored.

13. Colored dental ceramic according to at least one of claims 1 to 12, **characterized in that** the dental ceramics are fired after coloring.

14. Colored dental ceramic according to at least one of claims 1 to 13, **characterized in that** dental ceramics are crowns or bridges.

## Revendications

1. Céramique dentaire colorée pouvant être obtenue par le biais d'un procédé pour la coloration de céramiques dentaires à l'état poreux ou absorbant, **caractérisée en ce que** les céramiques dentaires sont translucides et sont utilisées pour la coloration de solutions d'ions métalliques ou de solutions de complexes métalliques.

2. Céramique dentaire colorée selon la revendication 1, **caractérisée en ce que** les solutions contiennent au moins un des ions ou complexes d'éléments de terres rares ou des groupes secondaires.

3. Céramique dentaire colorée selon la revendication 2, **caractérisée en ce que** les solutions contiennent du Pr, Er, Fe, Co, Ni ou Cu.

4. Céramique dentaire colorée selon la revendication 2 ou 3, **caractérisée en ce que** les solutions contiennent des sels et **en ce qu'**elles sont utilisées comme chlorures de sels, acétate ou alcools ainsi que complexes OXO.

5. Céramique dentaire colorée selon au moins une des revendications 1 à 4, **caractérisée en ce que** les céramiques dentaires sont utilisées dans l'état pré-fritté.

6. Céramique dentaire colorée selon au moins une des revendications 1 à 5, **caractérisée en ce que** des céramiques dentaires à base d'oxyde de zirconium ou d'oxyde d'aluminium sont utilisées.

7. Céramique dentaire colorée selon au moins une des revendications 1 à 6, **caractérisée en ce que** les solutions ioniques ou contenant des complexes sont à base d'eau ou d'alcool.

8. Céramique dentaire colorée selon au moins une des revendications 1 à 7, **caractérisée en ce que** la durée d'action des solutions ioniques ou contenant des complexes se trouve dans une plage horaire, notamment de moins de 2 heures, tout particulièrement de moins d'une heure et encore plus préférablement de moins de 20 minutes.

9. Céramique dentaire colorée selon au moins une des revendications 1 à 8, **caractérisée en ce que** la concentration des solutions est comprise entre 0,001 et 15 % en poids.

10. Céramique dentaire colorée selon au moins une des revendications 1 à 9, **caractérisée en ce que** la coloration est effectuée par immersion de la céramique dentaire dans les solutions, par application des solutions sur la céramique dentaire à l'aide d'instruments d'application ou par pulvérisation des solutions sur la céramique dentaire.

11. Céramique dentaire colorée selon au moins une des revendications 1 à 10, **caractérisée en ce que** les épaisseurs de paroi des céramiques dentaires à colorer présentent un diamètre de jusqu'à 10 mm, de préférence jusqu'à 7 mm.

12. Céramique dentaire colorée selon au moins une des revendications 1 à 11, **caractérisée en ce que** les céramiques dentaires sont entièrement colorées.

13. Céramique dentaire colorée selon au moins une des revendications 1 à 12, **caractérisée en ce que** les céramiques dentaires sont cuites après la coloration.

14. Céramique dentaire colorée selon au moins une des revendications 1 à 13, **caractérisée en ce que** les céramiques dentaires sont des couronnes ou des bridges.
